# FASCICULE DE BREVET EUROPEEN

(11) **EP 1 262 169 B1**
(45) Date de publication et mention de la délivrance du brevet: **02.10.2013**
(21) Numéro de dépôt: 02291298.4
(22) Date de dépôt: 28.05.2002
(51) Int. Cl.: A61K 8/73, A61K 8/64, A61K 8/34, A61K 38/18, A61K 36/81, A61K 38/04, A61K 38/22, A61K 31/00, A61K 31/165, A61K 31/726, A61K 31/728, A61K 36/00, A61K 36/67, A61Q 19/08, A61K 8/97, A61K 31/197, A61K 31/765

(54) **Composition pour le traitement des signes cutanés du vieillissement**
Hautalterungsschutzmittel
Skin anti-ageing composition

(30) Priorité: 29.05.2001 FR 0106979
(43) Date de publication de la demande: 04.12.2002
(73) Titulaire: L'Oréal, 75008 Paris (FR)
(72) Inventeur: De Lacharriere, Olivier, 75015 Paris (FR); Nouveau, Stéphanie, 92100 Boulogne (FR)
(74) Mandataire: Allab, Myriam

(56) Documents cités:
- EP-A- 0 217 413
- EP-A- 0 236 218
- EP-A- 0 444 638
- EP-A- 0 672 420
- EP-A- 0 797 984
- EP-A- 1 000 618
- WO-A-00/47040
- WO-A-00/47221
- WO-A-01/30375
- WO-A-95/13087
- WO-A-97/30690
- WO-A-97/35560
- WO-A-98/25643
- WO-A-98/48002
- WO-A-99/03448
- WO-A-99/38536
- US-A- 5 614 489
- US-A- 6 063 757
- US-B1- 6 172 086
- DATABASE WPI Week 198910 Derwent Publications Ltd., London, GB; AN 1989-071645 XP002272686 & JP 01 022809 A (KANEBO LTD), 25 janvier 1989 (1989-01-25)
- DATABASE WPI Week 199401 Derwent Publications Ltd., London, GB; AN 1994-002221 XP002272687 & JP 05 310548 A (TAISHO PHARM. CO. LTD.), 22 novembre 1993 (1993-11-22)
- DATABASE WPI Week 199822 Derwent Publications Ltd., London, GB; AN 1998-249300 XP002272688 & RU 2 092 152 C (BIOPREPARART CONCERN), 10 octobre 1997 (1997-10-10)
- DATABASE WPI Week 199817 Derwent Publications Ltd., London, GB; AN 1998-189185 XP002272689 & JP 10 045615 A (ICHIMARU PHARCOS INC.), 17 février 1998 (1998-02-17)
- DATABASE WPI Week 199531 Derwent Publications Ltd., London, GB; AN 1995-237128 XP002272690 & JP 07 145033 A (KOSE KK), 6 juin 1995 (1995-06-06)
- DATABASE WPI Week 200016 Derwent Publications Ltd., London, GB; AN 2000-176892 XP002272691 & JP 2000 026301 A (INAMURA KK), 25 janvier 2000 (2000-01-25)

## Description

L'invention a trait à un neuro-stimulant et/ou un neuro-activateur des nerfs sensitifs cutanés selon la revendication 1.

L'invention a également trait à l'utilisation d'un neuro-stimulant et/ou d'un neuro-activateur, pour augmenter l'épaisseur de l'épiderme.

Chez les mammifères en général, particulièrement chez l'homme, la peau est constituée de deux compartiments à savoir un compartiment profond qui sert de soutien, le derme, et un compartiment en relation avec l'extérieur, l'épiderme.

L'épiderme naturel est composé principalement de trois types de cellules qui sont les kératinocytes, très majoritaires, les mélanocytes et les cellules de Langerhans. Chacun de ces types cellulaires contribue par ses fonctions propres au rôle essentiel joué dans l'organisme par la peau.

Le derme fournit à l'épiderme un support solide. C'est également son élément nourricier. Il est principalement constitué de fibroblastes et d'une matrice extracellulaire composée elle-même principalement de collagène, d'élastine et d'une substance, dite substance fondamentale, composants synthétisés par le fibroblaste. On y trouve aussi des leucocytes, des mastocytes ou encore des macrophages tissulaires. Il est également traversé par des vaisseaux sanguins et des fibres nerveuses qui constituent ainsi le système nerveux cutané.

Le système nerveux cutané possède des fonctions sensorielle et neurovégétative. La fonction sensorielle est médiée par des récepteurs qui vont transformer les stimuli reçus en influx. Ces influx vont rejoindre le centre nerveux sensitif de la moelle épinière (ganglion rachidien) puis le cerveau où l'information est intégrée. Parmi les récepteurs, on trouve des mécanorécepteurs, des thermorécepteurs et des récepteurs nociceptifs. La fonction neurovégétative permet l'innervation des vaisseaux dermiques, des glandes sudoripares et des muscles arrecteurs des poils.

L'innervation cutanée permet de moduler les fonctions immunitaires par l'intermédiaire des neurotransmetteurs. Ainsi, compte tenu de sa distribution ubiquitaire, l'innervation cutanée est impliquée dans de nombreuses fonctions physiologiques (thermorégulation, vasomotricité, immunomodulation ou inflammation neurogène)(Pincelli C, Fantini F, Giannetti A. Neuropeptides and skin inflammation. Dermatology 1993,187 : 153-8).

L'innervation cutanée sensitive constitue la plus grande partie du réseau dermique et épidermique. La majorité des fibres nerveuses sensitives sont entourées par des cellules de Schwann. Ces fibres sont de type A delta (myélinisées) ou de type C (non myélinisées).
Les fibres nerveuses intra épidermiques sont distribuées jusqu'aux couches les plus superficielles de l'épiderme. Elles ont pour origine des faisceaux nerveux du derme supérieur et apparaissent comme des terminaisons libres. Ce sont des ramifications très fines qui peuvent monter directement dans les couches superficielles de l'épiderme ou selon un parcours plus sinueux entre les kératinocytes en perdant leur gaine de myéline et leurs cellules de Schwann.

Récemment, une étude ultra-structurale couplée à des marquages immunohistochimiques a confirmé l'existence de terminaisons nerveuses dans l'épiderme. Ces structures sont retrouvées dans les espaces intercellulaires de toutes les couches vivantes de l'épiderme, jusqu'aux plus superficielles. Elles établissent des contacts avec les corps cellulaires et les prolongements cytoplasmiques des kératinocytes par apposition de type «membrane à membrane », mais sans qu'aucune structure d'apparence synaptique ne soit observée (Hilliges M ; Wang L, Johansson O. Ultrastructural evidence for nerve fibers within all vital layers of the human epidermis. J Invest Dermatol 1995,104:134-7).

Les neuromédiateurs exercent des effets paracrines et autocrines sur les cellules cutanées. Dans l'autre sens, ils sont les vecteurs de l'information nerveuse depuis la périphérie jusqu'au système nerveux central. Ils permettent au système nerveux d'être informé en permanence des évènements qui surviennent à la périphérie (sensations cutanées) et ainsi en réponse de contrôler les principales fonctions physiologiques de la peau.

Les kératinocytes possèdent des récepteurs de la substance P (Staniek V, Misery L, Peguet-Navarro J, Abello J, Doutremepuich J, Claudy A, Schmitt D. Binding and in vitro modulation of human epidermal Langerhans cell functions by substance P. Arch Dermatol Res 1997,289 : 285-91), du Vasoactive intestinal peptide (VIP), du Calcitonin gene related peptide (CGRP) et du neuropeptide Y (Takahashi K, et al., Direct effects of cutaneous neuropeptides on adenylyl cyclase activity and proliferation in a keratinocyte cell line : stimulated cyclic AMP formation by CGRP and VIP/PHM, and inhibited NPY through G protein-couplked receptors. J Invest Dermatol 1993, 101:646-51), du Gastrin-releasing peptide (GRP) (Staniek V et al., Expression of Gastrin-releasing peptide receptor in human skin. Acta Derm Venereol 1996, 76 :282-6) et de l'acétylcholine (muscariniques ou nicotiniques)(Grando SA, et al., Keratinocyte muscarinic acetylcholine receptors: immunolocalization and partial characterization. J. invest. Dermatol., 1995, 104 : 95-100).

Des méthodes immunohistochimiques ont démontré que le récepteur du facteur de croissance des cellules nerveuses (NGF), est exprimé sur les kératinocytes basaux de la peau humaine normale. Les ARN messagers des récepteurs de haute et de faible affinité pour le NGF, ont été détectés dans des kératinocytes humains en culture (Pincelli C, et al., Expression and function of nerve growth factor receptor on cultured keratinocytes. J Invest Dermatol 1994, 103 : 13-8). Le NGF induit la prolifération des kératinocytes plutôt que leur différenciation. Cette prolifération est plus importante que celle provoquée par le facteur de croissance épidermique (EGF). Une fonction autocrine du NGF a pu être mise en évidence puisque les kératinocytes sont également capables de synthétiser et de sécréter du NGF (Pincelli, Expression and function of nerve growth factor receptor on cultured keratinocytes.J Invest Dermatol 1994, 103 : 13-8).

EP 1 000 618 décrit des compositions topiques de capsaicine améliorées en ce qu'elles limitent les effets secondaires indésirables liés à l'application topique de capsaicine, ces compositions sont utiles pour le traitement de pathologies cutanées douloureuses et de dysfonctionnements neuronaux.

RU2092152 décrit une lotion pour le visage ayant un effet hydratant et tenseur comprenant, entre autre, une fraction non saponifiée d'un extrait de *Capsicum annuum L.*

La demande japonaise JP 10-045615 décrit une préparation pour traitement externe stimulatrice de fibroblastes comprenant notamment des extraits de graines de *Capsicum annuum L*, cette préparation a pour utilité la prévention du vieillissement de la peau et pour sa bonne cicatrisation.

JP 7-145033 décrit une composition comprenant, parmi de nombreux extraits, un extrait de *Capsicum annuum Linne,* la composition étant destinée à prévenir le vieillissement de la peau et en éviter les rugosités.

On sait qu'au cours du vieillissement chronobiologique, l'épaisseur de l'épiderme se réduit. Les divisions cellulaires de la couche basale diminuent en nombre. Le temps de renouvellement des cellules cornées se rallonge. La maturation de ces cellules est imparfaite et la kératinisation n'aboutit plus à créer une couche cornée régulière et homogène.
On sait aussi que dans le cas de certaines maladies comme l'ichtyose la peau subit des dommages par défaut de prolifération cellulaire.

On sait aussi qu'à la ménopause le vieillissement cutané s'accélère, que l'épaisseur de la peau diminue, que les femmes se plaignent de ce que leur peau tire et qu'elle prend l'aspect d'une "peau sèche", voire de l'apparition d'une xérose. On sait que les déficits hormonaux associés à la ménopause s'accompagnent d'un ralentissement général du métabolisme cellulaire, d'où on peut quand même supposer que les effets que ressent la femme sont liés notamment à une diminution de la prolifération des kératinocytes.

La conséquence est donc qu'au cours du vieillissement la peau s'affine. La peau étant la barrière naturelle aux agressions venant de l'extérieur, il est logique de penser que plus la peau est épaisse, plus la sensibilité de celle-ci devrait diminuer (effet barrière important). A l'inverse, plus la peau est fine plus sa sensibilité devrait être augmentée (accessibilité aux terminaisons nerveuse accrue).

Or la demanderesse vient maintenant de montrer que contrairement aux idées reçues, plus l'épiderme est fin plus la sensibilité nerveuse diminue.

Ainsi la diminution de l'épaisseur de l'épiderme observée au cours du vieillissement est corrélée à une diminution de l'innervation cutanée, cette dernière ayant pour conséquence une diminution de l'apport en neuromédiateurs et/ou facteurs de croissance nerveux qui pourrait entraîner une diminution, voire un arrêt, de la prolifération des cellules de l'épiderme, particulièrement des kératinocytes.

Aussi la demanderesse propose-t-elle de lutter contre la diminution de l'épaisseur de la peau liée au vieillissement, particulièrement de l'épaisseur de l'épiderme, en appliquant sur la peau une composition comprenant un neuro-stimulant, et/ou neuro-activateur des nerfs sensitifs cutanés.

Par neuro-stimulant et/ou neuro-activateur on entend une substance qui accroît l'activité physiologique des nerfs.

Ainsi, l'invention vise à maintenir par la fourniture des substances précitées une bonne vitalité, une bonne intégrité, une bonne activité, une bonne prolifération, un bon fonctionnement et/ou une bonne excitabilité aux nerfs cutanés, particulièrement aux nerfs sensitifs cutanés.

Comme neuro-stimulant et/ou neuro-activateur, on peut citer par exemple les capsicosides, les capsidiols, la capsaxanthine, les capsaïcinoïdes comme la capsaïcine, ou des extraits végétaux en contenant comme ceux de paprika *(Capsicum annuum),* de piment rouge (*Red pepper*), ou de poivre (*Piper nigrum*).

Préférentiellement, la composition de l'invention contient des capsaïcinoïdes ou des extraits végétaux en contenant encore plus préférentiellement de la capsaïcine.

Bien évidemment les neuro-stimulants et/ou les neuro-activateurs peuvent être utilisés dans les compositions de l'invention seuls ou en mélange et en toute proportion.

La quantité de neuro-stimulants et/ou de neuro-activateurs comprise dans la composition selon l'invention est bien entendu fonction de l'effet recherché et peut donc varier dans une large mesure.
Pour donner un ordre de grandeur, dans la composition selon l'invention le facteur de croissance et/ou le neuro-stimulant et/ou le neuro-activateur peuvent être en une quantité représentant de 10⁻⁸% à 5% du poids total de la composition et préférentiellement en une quantité représentant de 10⁻⁵% à 0,5% du poids total de la composition.

La composition de l'invention peut être sous toutes formes galéniques imaginables, adaptée aussi bien à une application topique sur la peau et/ou les muqueuses et/ou les cheveux et/ou les ongles qu'à une administration par la voie orale.

De manière préférentielle, la composition de l'invention est destinée à une administration par la voie topique sur la peau.

La composition de l'invention peut être une composition cosmétique ou dermatologique. Préférentiellement selon l'invention, la composition est une composition cosmétique. La composition est une composition cosmétique car elle est destinée à améliorer l'aspect cutané général de l'individu qui en fait usage.

Très préférentiellement la composition de l'invention est une composition cosmétique destinée à une administration par la voie topique.

Pour une administration par la voie orale, la composition de l'invention peut se présenter sous toutes les formes adaptées, particulièrement sous forme d'une solution buvable, d'un sirop, d'un comprimé, d'une dragée, d'une gélule, d'une capsule ou encore d'un aliment nutritionnel ou d'un complément nutritionnel.
Ladite composition peut comprendre en outre au moins un excipient approprié adapté à l'administration orale.

Pour une administration par application topique sur la peau, les cheveux et/ou les muqueuses, la composition selon l'invention comprend bien évidemment un support cosmétiquement acceptable, c'est à dire un support compatible avec la peau, les muqueuses, les ongles, les cheveux et peut se présenter sous toutes les formes galéniques normalement utilisées pour une application topique, notamment sous forme d'une solution aqueuse, hydroalcoolique ou huileuse, d'une émulsion huile-dans-eau ou eau-dans-huile ou multiple, d'un gel aqueux ou huileux, d'un produit anhydre liquide, pâteux ou solide, d'une dispersion d'huile dans une phase aqueuse à l'aide de sphérules, ces sphérules pouvant être des nanoparticules polymériques telles que les nanosphères et les nanocapsules ou mieux des vésicules lipidiques de type ionique et/ou non-ionique.

Cette composition peut être plus ou moins fluide et avoir l'aspect d'une crème blanche ou colorée, d'une pommade, d'un lait, d'une lotion, d'un sérum, d'une pâte, d'une mousse. Elle peut éventuellement être appliquée sur la peau sous forme d'aérosol. Elle peut également se présenter sous forme solide, et par exemple sous forme de stick. Elle peut être utilisée comme produit de soin, comme produit de nettoyage, comme produit de maquillage ou encore comme simple produit déodorant.

De façon connue, la composition de l'invention peut contenir les adjuvants habituels dans les domaines cosmétique et dermatologique, tels que les gélifiants hydrophiles ou lipophiles, les actifs hydrophiles ou lipophiles, les conservateurs, les antioxydants, les solvants, les parfums, les charges, les filtres, les pigments, les agents chélateurs, les absorbeurs d'odeur et les matières colorantes. Les quantités de ces différents adjuvants sont celles classiquement utilisées dans les domaines considérés, et par exemple de 0,01% à 20% du poids total de la composition. Ces adjuvants, selon leur nature, peuvent être introduits dans la phase grasse, dans la phase aqueuse, dans les vésicules lipidiques et/ou dans les nanoparticules.

Lorsque la composition de l'invention est une émulsion, la proportion de la phase grasse peut aller de 5% à 80% en poids, et de préférence de 5% à 50% du poids total de la composition. Les huiles, les émulsionnants et les coémulsionnants utilisés dans la composition sous forme d'émulsion sont choisis parmi ceux classiquement utilisés dans le domaine considéré. L'émulsionnant et le coémulsionnant sont présents, dans la composition, en une proportion allant de 0,3% à 30% en poids, et de préférence de 0,5% à 20% du poids total de la composition.

Comme huiles utilisables dans l'invention, on peut citer les huiles minérales, les huiles d'origine végétale (huile d'abricot, huile de tournesol), les huiles d'origine animale, les huiles de synthèse, les huiles siliconées et les huiles fluorées (perfluoropolyéthers). On peut aussi utiliser comme matières grasses des alcools gras (alcool cétylique), des acides gras, des cires (cire d'abeilles).

Comme émulsionnants et coémulsionnants utilisables dans l'invention, on peut citer par exemple les esters d'acide gras et de polyéthylène glycol tels que le stéarate de PEG-40, le stéarate de PEG-100, les esters d'acide gras et de polyol tels que le stéarate de glycéryle et le tristéarate de sorbitane.

Comme gélifiants hydrophiles, on peut citer en particulier les polymères carboxyvinyliques (carbomer), les copolymères acryliques tels que les copolymères d'acrylates/alkylacrylates, les polyacrylamides, les polysaccharides, les gommes naturelles et les argiles, et, comme gélifiants lipophiles, on peut citer les argiles modifiées comme les bentones, les sels métalliques d'acides gras, la silice hydrophobe et les polyéthylènes.

La composition peut contenir d'autres actifs hydrophiles comme les protéines ou les hydrolysats de protéine, les acides aminés, les polyols, l'urée, l'allantoïne, les sucres et les dérivés de sucre, les extraits végétaux et les hydroxy-acides.

Comme actifs lipophiles, on peut utiliser le rétinol (vitamine A) et ses dérivés, l'α-tocophérol (vitamine E) et ses dérivés (esters, sels, etc.), les acides gras essentiels, les céramides, les huiles essentielles, l'acide salicylique et ses dérivés ou encore les vitamines B1, B6 et /ou B12.

Il est également possible d'utiliser dans la composition de l'invention la vitamine C (ou acide ascorbique) et ses dérivés (esters, sels, etc.).

On peut également y ajouter un extrait végétal riche en isoflavonoïdes, comme par exemple l'extrait de soja (Glycina max) disponible auprès de Archer Daniels Midland Company sous la dénomination Novasoy®.

Il est enfin possible d'ajouter à la composition de l'invention un progestatif comme par exemple la 17-hydroxyprogestérone ou la prégnénolone.

La figure 1 montre les valeurs moyennes de l'épaisseur de l'épiderme pour différents seuils de détection au froid.
La figure 2 montre le seuil de détection thermique (chaud et froid) en fonction du groupe d'âge.
La figure 3 montre les valeurs moyennes des scores totaux obtenus dans le test à la capsaïcine pour les différentes classes d'âge.

Les exemples et compositions suivants illustrent l'invention sans la limiter aucunement. Dans les compositions les proportions indiquées sont des pourcentages en poids.

### Exemple 1 : Corrélation entre la baisse de sensibilité thermique cutanée et l'épaisseur de l'épiderme mesurée chez les sujets âgés :

### Méthode :

- Nombre de sujets : 122 femmes âgées de 60 à 80 ans
- Mesure des seuils de détection cutanée du froid :
   Cette mesure se fait avec le Thermal Sensory Analyser (TSA 2001 Medoc Ltd, Ramat Yishai, Israël) dont la sonde est capable par effet Peltier de refroidir la peau selon un gradient calibré de température.
   On détecte ainsi le seuil de sensibilité au froid à partir d'une température "neutre" pour la peau de 32°C. Il suffit au sujet de signaler par l'intermédiaire d'un bouton poussoir dès qu'il ressent un changement de température, geste qui entraîne automatiquement l'arrêt du stimulus thermique.
   Une série de 5 stimuli (inférieurs à 32°C) est enregistrée et une moyenne est calculée pour déterminer le seuil de détection. Cette mesure est faite sur le visage.
- Mesure de l'épaisseur de l'épiderme :
   L'épaisseur de l'épiderme est mesurée en échographie ultrasonore à haute résolution (25MHZ) sur le dos de la main (Querleux B., Lévêque JL, de Rigal J : In vivo cross sectional ultrasonic imaging of human skin. Dermatologica 1988, 77: 332-337.).

### Résultats :

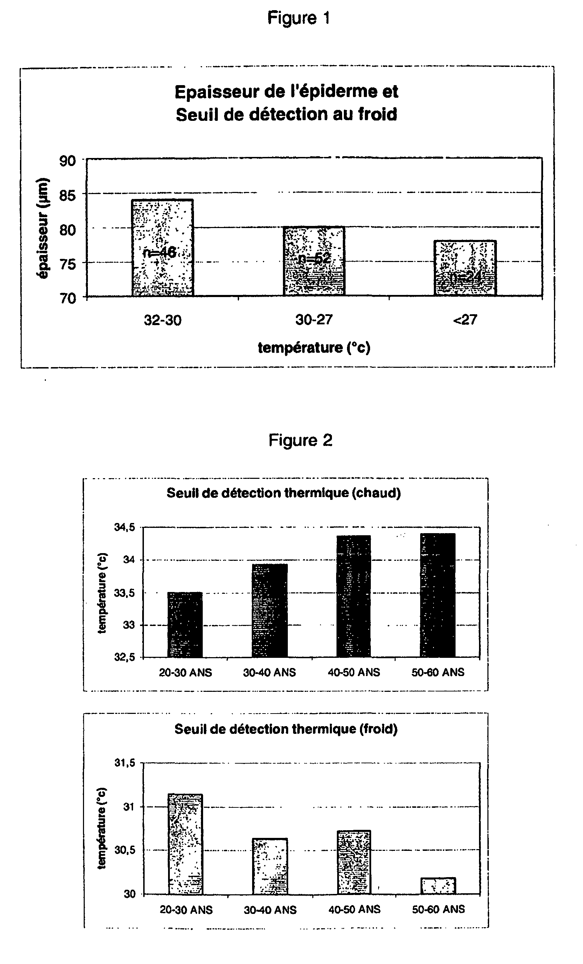

Les valeurs moyennes de l'épaisseur de l'épiderme pour différents seuils de détection au froid sont présentées dans la figure 1.
Plus la température de détection est élevée, plus la peau est sensible au froid et plus l'épiderme est épais, Au contraire, moins la peau est sensible au froid, plus l'épiderme est atrophié.

### Exemple 2 : Mesures de l'acuité thermique en fonction de l'âge :

### Méthode :

- Nombre de sujets : 160 femmes classées en 4 groupes de 40 :
   1. 40 sujets de 20 à 30 ans,
   2. 40 sujets de 30 à 40 ans,
   3. 40 sujets de 40 à 50 ans
   4. 40 sujets de 50 à 60 ans
- Mesure des seuils de détection cutanée au chaud et au froid (sensibilité thermique) :
   Cette mesure est faite selon le protocole décrit à l'exemple 1 sur la lèvre supérieure (partie cutanée) en zone latérale droite ou gauche selon le plan de randomisation.

### Résultats : (figure 2)

Plus les sujets sont âgés, moins ils sont sensibles à la température (diminution des seuils de détection du froid).

### Exemple 3 : Mesures de la sensibilité à la capsaïcine en fonction de l'âge

### Méthode :

Sujets : 152 femmes âgées de 18 à 65 ans : 35 ont moins de 30 ans ; 32 ont entre 30 et 40 ans ; 43 ont entre 41 et 50 ans ; 20 ont entre 51 et 60 ans ; 21 ont plus de 60 ans.

### Test à la capsaïcine :

Le test à la capsaïcine, utilise un composé naturel de la famille des alcaloïdes présent dans les fruits de différentes espèces de la famille de la belladone. La capsaïcine est la substance piquante présente dans les piments. L'application de capsaïcine sur la peau entraîne une brève libération de substance P au niveau des terminaisons nerveuses avec comme conséquence l'apparition de sensations de brûlures et de picotements accompagnées parfois d'un érythème localisé à la région d'application. Ces réactions sont transitoires et totalement réversibles au cours des heures qui suivent. Ces réactions sont directement liées à l'innervation sensitive ; plus celle-ci est importante, plus la réaction est importante et inversement.
- application de 20 mg/cm² de Zostrix HP (crème à 0.075% de capsaïcine) sur un site de 4 cm² à l'angle de la mâchoire.
- Cotations (échelle à 4 niveaux) par le sujet des sensations de picotements, d'échauffements et démangeaisons à 3mn, 5mn, 10mn, 15mn, 20mn, 25mn et 30mn après l'application de capsaïcine ressenties selon l'échelle suivante :
   0 = Absence de réaction ;
   1 = Sensation légère ou douteuse ;
   2 = Sensation moyenne ou franche ;
   3 = Sensation forte ou importante.
- Evaluations cliniques de l'érythème à 3mn, 5mn, 15mn, 20mn, 25mn et 30mn par l'investigateur selon l'échelle suivante :
   0 = Absence de réaction ;
   1 = Erythème léger ou douteux ;
   2 = Erythème modéré localisé à la zone d'application ;
   3 = Erythème important avec extension au-delà des limites de la zone d'application.

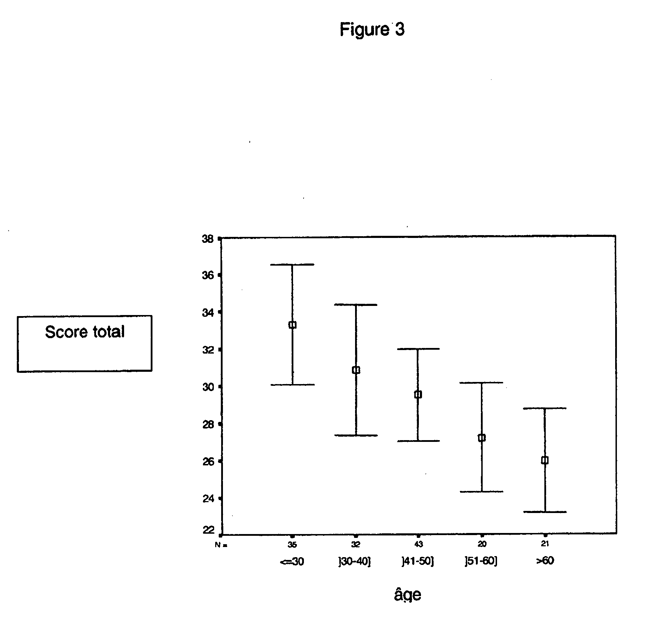

### Résultats (voir figure 3) :

Ces résultats montrent une nette diminution des scores pour les différentes classes d'âge.

Les trois exemples précédents montrent bien une diminution de la sensibilité cutanée corrélée à une diminution de l'épaisseur de l'épiderme au cours du vieillissement. Le premier exemple montre de plus une diminution de l'épaisseur de l'épiderme corrélée à une perte de la sensibilité au froid.

### Exemple 4 : Exemples de formulations illustrant l'invention et particulièrement les compositions selon l'invention. Ces compositions ont été obtenues par simple mélange des différents composants.

| Composition 1 : Crème pour la peau : | |
|---|---|
| Capsaïcine | 0,0005 |
| Stéarate de glycérol | 2,00 |
| Polysorbate 60 (Tween 60 vendu par la société ICI) | 1,00 |
| Acide stéarique | 1,40 |
| Acide glycyrrhétinique | 2,00 |
| Triéthanolamine | 0,70 |
| Carbomer | 0,40 |
| Fraction liquide du beurre de karité | 12,00 |
| Huile de tournesol | 10,00 |
| Antioxydant | 0,05 |
| Parfum | 0,5 |
| Conservateur | 0,30 |
| Eau | qsp 100% |

| Composition 2 : Crème pour la peau : | |
|---|---|
| Capsaïcine | 0,0001 |
| 17-OH progestérone ou acétate de prégnénolone | 1 |
| Stéarate de glycérol | 2,00 |
| Polysorbate 60 (Tween 60 vendu par la société ICI) | 1,00 |
| Acide stéarique | 1,40 |
| Acide glycyrrhétinique | 2,00 |
| Triéthanolamine | 0,70 |
| Carbomer | 0,40 |
| Fraction liquide du beurre de karité | 12,00 |
| Huile de tournesol | 10,00 |
| Antioxydant | 0,05 |
| Parfum | 0,5 |
| Conservateur | 0,30 |
| Eau | qsp 100% |

| Composition 3 : Crème pour la peau | |
|---|---|
| Extrait de *Capsicum annuum* | 0,005 |
| Stéarate de glycérol | 2,00 |
| Polysorbate 60 (Tween 60 vendu par la société ICI) | 1,00 |
| Acide stéarique | 1,40 |
| Acide glycyrrhétinique | 2,00 |
| Triéthanolamine | 0,70 |
| Carbomer | 0,40 |
| Fraction liquide du beurre de karité | 12,00 |
| Huile de tournesol | 10,00 |
| Antioxydant | 0,05 |
| Parfum | 0,5 |
| Conservateur | 0,30 |
| Eau | qsp 100% |

## Revendications

1. Neuro-stimulant et/ou neuroactivateur choisi parmi les capsicosides, les capsidiols, la capsaxanthine, les capsaïcinoïdes comme la capsaïcine, ou des extraits végétaux en contenant comme ceux de paprika (*Capsicum annuum*), de piment rouge (*Red pepper*), ou de poivre (*Piper nigrum*), pour maintenir la vitalité, l'intégrité, l'activité, la prolifération, le fonctionnement et/ou l'excitabilité aux nerfs sensitifs cutanés.

2. Neuro-stimulant et/ou neuroactivateur choisi parmi les capsicosides, les capsidiols, la capsaxanthine, les capsaïcinoïdes comme la capsaïcine, ou des extraits végétaux en contenant comme ceux de paprika (*Capsicum annuum*), de piment rouge (*Red pepper*), ou de poivre (*Piper nigrum*), pour maintenir et/ou augmenter l'épaisseur de l'épiderme.

3. Neuro-stimulant et/ou neuroactivateur pour une utilisation selon la revendication 1 ou 2, **caractérisé en ce qu'**il est un capsaïcinoïde ou un extrait végétal en contenant.

4. Neuro-stimulant et/ou neuroactivateur pour une utilisation selon la revendication 3, **caractérisé en ce que** le capsaïcinoïde est la capsaïcine.

5. Neuro-stimulant et/ou neuroactivateur pour une utilisation selon la revendication 3, **caractérisé en ce que** l'extrait végétal est un extrait de paprika (*Capsicum annuum*), de piment rouge (*Red pepper*), ou de poivre (*Piper nigrum*).

6. Neuro-stimulant et/ou neuroactivateur pour une utilisation selon l'une quelconque des revendications précédentes, **caractérisé par le fait qu'**il est dans une composition en une quantité représentant de 10⁻⁸% à 5% du poids total de la composition.

7. Neuro-stimulant et/ou neuroactivateur pour une utilisation selon la revendication précédente, **caractérisé par le fait qu'**il est dans une composition en une quantité représentant de 10⁻⁵% à 0,5% du poids total de la composition.

8. Neuro-stimulant et/ou neuroactivateur pour une utilisation selon l'une quelconque des revendications précédentes, **caractérisé par le fait que** la composition est adaptée à une application topique sur la peau.

9. Utilisation cosmétique non-thérapeutique d'au moins un neuro-stimulant et/ou neuroactivateur choisi parmi les capsicosides, les capsidiols, la capsaxanthine, les capsaïcinoïdes comme la capsaïcine, ou des extraits végétaux en contenant comme ceux de paprika (*Capsicum annuum*), de piment rouge (*Red pepper*), ou de poivre (*Piper nigrum*), pour maintenir et/ou augmenter l'épaisseur de l'épiderme de façon à lutter contre la diminution de l'épaisseur de la peau liée au vieillissement.

## Patentansprüche

1. Neurostimulans und/oder Neuroaktivator, ausgewählt aus den Capsicosiden, den Capsidiolen, Capsaxanthin, den Capsaicinoiden, wie Capsaicin, oder Pflanzenextrakten, die diese enthalten, wie diejenigen aus Paprika (*Capsicum annuum*), Cayennepfeffer (*Red* Pepper) oder Pfeffer (*Piper nigrum*), um die Vitalität, die Unversehrtheit, die Aktivität, die Proliferation, die Funktion und/oder die Erregbarkeit der sensorischen Nerven der Haut zu erhalten.

2. Neurostimulans und/oder Neuroaktivator, ausgewählt aus den Capsicosiden, den Capsidiolen, Capsaxanthin, den Capsaicinoiden, wie Capsaicin, oder Pflanzenextrakten, die diese enthalten, wie diejenigen aus Paprika (*Capsicum annuum*), Cayennepfeffer (*Red Pepper*) oder Pfeffer (*Piper nigrum*), um die Dicke der Epidermis zu erhalten und/oder zu erhöhen.

3. Neurostimulans und/oder Neuroaktivator für die Verwendung nach Anspruch 1 oder 2, **dadurch gekennzeichnet, dass** es sich um ein Capsaicinoid oder einen Pflanzenextrakt handelt, der dieses enthält.

4. Neurostimulans und/oder Neuroaktivator für die Verwendung nach Anspruch 3, **dadurch gekennzeichnet, dass** es sich bei dem Capsaicinoid um Capsaicin handelt.

5. Neurostimulans und/oder Neuroaktivator für die Verwendung nach Anspruch 3, **dadurch gekennzeichnet, dass** der Pflanzenextrakt ein Extrakt aus Paprika (*Capsicum annuum*), Cayennepfeffer (*Red Pepper*) oder Pfeffer (*Piper nigrum*) ist.

6. Neurostimulans und/oder Neuroaktivator für die Verwendung nach einem der vorhergehenden Ansprüche, **dadurch gekennzeichnet, dass** er in einer Zusammensetzung in einer Menge vorliegt, die 10⁻⁸ % bis 5 %, bezogen auf das Gesamtgewicht der Zusammensetzung, ausmacht.

7. Neurostimulans und/oder Neuroaktivator für die Verwendung nach dem vorhergehenden Anspruch, **dadurch gekennzeichnet, dass** er in einer Zusammensetzung in einer Menge vorliegt, die 10⁻⁵ % bis 0,5 %, bezogen auf das Gesamtgewicht der Zusammensetzung, ausmacht.

8. Neurostimulans und/oder Neuroaktivator für die Verwendung nach einem der vorhergehenden Ansprüche, **dadurch gekennzeichnet, dass** die Zusammensetzung an eine topische Anwendung auf die Haut angepasst ist.

9. Nicht-therapeutische kosmetische Verwendung von mindestens einem Neurostimulans und/oder Neuroaktivator, ausgewählt aus den Capsicosiden, den Capsidiolen, Capsaxanthin, den Capsaicinoiden, wie Capsaicin, oder Pflanzenextrakten, die diese enthalten, wie diejenigen aus Paprika (*Capsicum annuum*), Cayennepfeffer (*Red Pepper*) oder Pfeffer (*Piper nigrum*), um die Dicke der Epidermis zu erhalten und/oder zu erhöhen und so die Verringerung der Dicke der Haut in Verbindung mit dem Altern zu bekämpfen.

## Claims

1. Neurostimulant and/or neuroactivator chosen from capsicoides, capsidiols, capsaxanthin, capsaicinoids such as capsaicin, or plant extracts containing same, such as those of paprika (*Capsicum annuum*), of red pepper or of pepper *(Piper nigrum*), for maintaining the vitality, integrity, activity, proliferation, functioning and/or excitability in the sensory nerves of the skin.

2. Neurostimulant and/or neuroactivator chosen from capsicoides, capsidiols, capsaxanthin, capsaicinoids such as capsaicin, or plant extracts containing same, such as those of paprika (*Capsicum annuum*), of red pepper or of pepper (*Piper nigrum*), for maintaining and/or increasing the thickness of the epidermis.

3. Neurostimulant and/or neuroactivator for use according to Claim 1 or 2, **characterized in that** it is a capsaicinoid or a plant extract containing same.

4. Neurostimulant and/or neuroactivator for use according to Claim 3, **characterized in that** the capsaicinoid is capsaicin.

5. Neurostimulant and/or neuroactivator for use according to Claim 3, **characterized in that** the plant extract is an extract of paprika (*Capsicum annuum*), of red pepper or of pepper (*Piper nigrum*).

6. Neurostimulant and/or neuroactivator for use according to any one of the preceding claims, **characterized in that** it is in a composition in an amount representing from 10⁻⁸% to 5% of the total weight of the composition.

7. Neurostimulant and/or neuroactivator for use according to the preceding claim, **characterized in that** it is in a composition in an amount representing from 10⁻⁵% to 0.5% by weight of the total weight of the composition.

8. Neurostimulant and/or neuroactivator for use according to any one of the preceding claims, **characterized in that** the composition is suitable for topical application to the skin.

9. Non-therapeutic cosmetic use of at least one neurostimulant and/or neuroactivator chosen from capsicoides, capsidiols, capsaxanthin, capsaicinoids such as capsaicin, or plant extracts containing same, such as those of paprika (*Capsicum annuum*), of red pepper or of pepper (*Piper nigrum*)*,* for maintaining and/or increasing the thickness of the epidermis so as to combat the decrease in thickness of the skin related to ageing.
